# EUROPÄISCHE PATENTANMELDUNG

(11) **EP 1 688 088 A1**
(43) Veröffentlichungstag der Anmeldung: **09.08.2006**
(21) Anmeldenummer: 06002500.4
(22) Anmeldetag: 07.02.2006
(51) Int. Cl.: A61B 5/00, G06F 19/00

(54) **Datenerfassungseinrichtung zur Erfassung von physiologischen Parametern**

(30) Priorität: 08.02.2005 DE 102005005632
(71) Anmelder: Actimon GmbH & Co. KG, 69190 Walldorf (DE)
(72) Erfinder: Wegertseder, Dominik, 85540 Haar (DE); Schweizer, Thomas, 85560 Ebersberg (DE)
(74) Vertreter: Müller-Boré & Partner Patentanwälte

(57) **Zusammenfassung**

Datenerfassungssystem zur Erfassung von physiologischen Parametern eines Lebewesens, umfassend:
- zumindest eine Sensoreinheit zur Erfassung zumindest eines physiologischen Parameters des Lebewesens und Erzeugung entsprechender Parameterdaten;
- zumindest ein Mittel zur Erfassung von mit dem physiologischen Parameter in Zusammenhang stehenden Zusatzdaten; und
- zumindest eine Verarbeitungseinheit mit einem Komparator zum Vergleich der Parameterdaten und der Zusatzdaten, wobei der Komparator ausgelegt ist, in Abhängigkeit des Vergleichsergebnisses ein Signal an eine Korrektureinheit der Verarbeitungseinheit zu übermitteln, um eine Korrektur von mit Fehlern versehenen Parameterdaten vorzusehen.

## Beschreibung

Die vorliegende Erfindung bezieht sich auf eine Datenerfassungseinrichtung zur Erfassung von physiologischen Parametern eines Lebewesens, auf ein Verfahren zur Erfassung von physiologischen Parametern eines Lebewesens sowie auf ein entsprechendes Computerprogrammprodukt.

Datenerfassungseinrichtungen zur Erfassung von physiologischen Parametern eines Lebewesens sind hinlänglich aus dem Stand der Technik bekannt. So existieren z.B. Vorrichtungen, wie ein EKG- oder Pulsmesser, welche entsprechende Parameter einer Person messen. Durch Bewegung der Lebewesen bei der Messung treten jedoch häufig Störungen, wie Artefakte, auf, welche zu einer Ungenauigkeit der Messung bzw. Auswertung der Daten führt. Insbesondere ist es hierbei schwierig zu ermitteln, ob lediglich eine Signalstörung in Form eines Artefakts vorliegt oder eine ernstzunehmende sonstige Störung der Signale, wie z.B. eine Fehlfunktion des Messgeräts oder eine falsche Bedienung. Zur Lösung dieses Problems wurden bisher geschulte Fachkräfte eingesetzt, welche während der Messung eine Prüffunktion ausübten, um Störungen entsprechend zu erkennen.

Es ist Aufgabe der vorliegenden Erfindung, eine Datenerfassungseinrichtung zur Erfassung von physiologischen Parametern eines Lebewesens, ein Verfahren zur Erfassung von physiologischen Parametern eines Lebewesens sowie ein entsprechendes Computerprogrammprodukt vorzusehen, mittels welchen physiologische Parameter des Lebewesens einfacher und sicherer gemessen werden können.

Diese Aufgabe wird durch eine Datenerfassungseinrichtung zur Erfassung von physiologischen Parametern eines Lebewesens gemäß Anspruch 1, ein Verfahren zur Erfassung von physiologischen Parametern eines Lebewesens gemäß Anspruch 10 sowie ein entsprechendes Computerprogrammprodukt gemäß Anspruch 11 gelöst. Bevorzugte Ausführungsformen sind Gegenstand der abhängigen Unteransprüche.

Erfindungsgemäß ist eine Datenerfassungseinrichtung zur Erfassung von physiologischen Parametern eines Lebewesens vorgesehen, umfassend:
- zumindest eine Sensoreinheit, welche ausgelegt ist, zumindest einen physiologischen Parameter des Lebewesens zu erfassen und entsprechende Parameterdaten zu erzeugen;
- zumindest ein Mittel bzw. eine Zusatzdatenerfassungseinheit, welche ausgelegt ist, mit dem physiologischen Parameter im Zusammenhang stehende Zusatzdaten zu erfassen; und
- zumindest eine Verarbeitungseinheit mit einem Komparator zum Vergleich der Parameterdaten und der Zusatzdaten, wobei der Komparator ausgelegt ist, in Abhängigkeit des Vergleichsergebnisses ein Signal an eine Korrektureinheit der Verarbeitungseinheit zu übermitteln, um eine Korrektur von mit Fehlern versehenen Parameterdaten vorzusehen.

Es wird somit eine Datenerfassungseinrichtung vorgesehen, welche wenigstens eine Sensoreinheit aufweist. Die Sensoreinheit ist im Wesentlichen am oder in räumlicher Nähe zu dem Lebewesen angeordnet, um entsprechend die physiologischen Parameter des Lebewesens zu erfassen. Insbesondere können Sensoren vorgesehen werden, welche die Beschleunigung, die Lage im Raum, die Drehrate, die Impedanz, die Feuchte (Hautfeuchte), den Widerstand (Hautwiderstand) und/oder die Temperatur erfassen. Darüber hinaus können Sensoren, welche den Sauerstoffsättigungsgehalt des Blutes, die Atmungsfrequenz, den Blutdruck oder ähnliche Größen erfassen, vorgesehen sein. Darüber hinaus ist denkbar, dass ebenfalls von externer Seite, d.h. durch das Lebewesen selbst, Daten der Sensoreinheit zugeführt werden können, wie z.B. der Gemütszustand oder Einnahmezeiten und/oder -mengen von Medikamenten. Der Sensor ist am Objekt bzw. Lebewesen oder in unmittelbarer räumlicher Nähe zu diesem angeordnet, wobei unter "unmittelbarer räumlicher Nähe" vorzugsweise ein Bereich bis zu ca. 20 m, bevorzugterweise bis ca. 10 m und ganz besonders bevorzugterweise bis ca. 1 m zu verstehen ist. Darüber hinaus ist zumindest die Sensoreinheit vorteilhafterweise mobil ausgebildet, so dass diese mit dem Lebewesen bewegbar ist. In anderen Worten ist "mobil" vorzugsweise dahingehend zu versehen, dass die räumlichen Abmessungen und das Gewicht so ausgelegt sind, dass die Sensoreinheit durch das Lebewesen bewegbar ist. Insbesondere ist die Sensoreinheit somit leichter als ca. 5 kg und ganz besonders bevorzugterweise leichter als ca. 1 kg. Die räumliche Ausdehnung der Sensoreinheit ist vorteilhafterweise geringer als 0,5 m³ und ganz bevorzugt geringer als ca. 0,1 m³. Die Mittel zur Erfassung der Zusatzdaten sind vorteilhafterweise in ihrer räumlichen Ausdehnung, ihrem Gewicht im wesentlichen gleich wie die Sensoreinheit ausgebildet. Darüber hinaus können die Mittel zur Erfassung der Zusatzdaten ebenfalls am Lebewesen angeordnet sein oder in unmittelbarer räumlicher Nähe. Die Mittel zur Erfassung von Zusatzdaten sind vorteilhafterweise ausgelegt, mit dem physiologischen Parameter im Zusammenhang stehende Daten zu erfassen. Die Zusatzdaten können einerseits gemessene Daten darstellen, andererseits jedoch auch vorgegebene Daten, wie z.B. bestimmte SollWerte von zu messenden Parametern (vorzugsweise über einen gewissen Zeitraum). Infolgedessen werden bei der erfindungsgemäßen Datenerfassungseinrichtung zur Erfassung eines physiologischen Parameters wenigstens zwei verschiedene Daten erfasst, nämlich die Parameterdaten sowie die Zusatzdaten. Vorteilhafterweise weist die Datenerfassungseinrichtung weiterhin eine Verarbeitungseinheit auf. Die Verarbeitungseinheit kann einerseits am Lebewesen bzw. in unmittelbarer räumlicher Nähe zu diesem angeordnet sein und mobil ausgebildet sein. Insbesondere vorteilhafterweise ist die Verarbeitungseinheit in kompakter Bauart ausgeführt und eigenständig am Lebewesen anordenbar, d.h. unabhängig von der Position der weiteren Elemente der Datenerfassungseinrichtung positionierbar. In einer bevorzugten Ausführungsform ist die Verarbeitungseinheit als Mobiltelefon oder PDA (Personal Digital Assistent) ausgebildet. Die Verarbeitungseinheit kann jedoch auch ortsfern angeordnet sein und über eine drahtgebundene oder drahtlose Signalverbindung mit der Sensoreinheit und dem Mittel zur Erfassung von Zusatzdaten in Verbindung stehen. Die Verarbeitungseinheit umfasst wenigstens einen Komparator und eine Korrektureinheit. Der Komparator ist vorteilhafterweise ausgelegt, die Parameterdaten und die Zusatzdaten zu erfassen und diese einem Vergleich miteinander zu unterziehen. Dies kann einerseits ein direkter Vergleich dieser sein oder auch eine indirekte Zuordnung der Zusatzdaten zu den Parameterdaten. Eine indirekte Zuordnung ist somit insbesondere so zu verstehen, dass in einem bestimmten Zeitpunkt erfasste Parameterdaten im gleichen Zeitpunkt erfasste Zusatzdaten zugeordnet werden. Bei einer Messung über einen längeren Zeitraum werden somit im wesentlichen parallel Parameterdaten und Zusatzdaten erfasst, so dass sich eine Abhängigkeit zwischen diesen beiden ergibt. Vorteilhafterweise ist die Verarbeitungseinheit ausgebildet, aus den parallel gemessenen Parameterdaten und Zusatzdaten ein Datenmuster zu erstellen. Aufgrund des Datenmusters bzw. der Abhängigkeit von Parameterdaten zu Zusatzdaten ist die Korrektureinheit ausgelegt, ein Signal an weitere in der oder ausserhalb der Verarbeitungseinheit vorgesehene Mittel zu übermitteln. Sollten z.B. die Parameterdaten aufgrund eines Artefakts gestört bzw. verändert sein, so verändern sich auch entsprechend die Zusatzdaten, so dass über das Signal an die Verarbeitungseinheit dieser angezeigt wird, dass ein Artefakt vorliegt. Infolgedessen ist es möglich, eine Korrektur der entsprechenden Parameterdaten vorzusehen. Somit wird vorteilhafterweise eine Datenerfassungseinrichtung geschaffen, bei welcher Störungen, insbesondere Artefakte, zuverlässig erkannt werden können, wobei eine Überwachung durch eine geschulte Fachkraft nicht mehr notwendig ist. Darüber hinaus werden diese Artefakte vorteilhafterweise durch die Datenerfassungseinrichtung korrigiert.

Bevorzugterweise sind die Mittel zur Erfassung von Zusatzdaten ausgelegt, andere als die durch die Sensoreinheit erfassten Parameter, insbesondere andere physiologische Parameter, zu erfassen. So ist es beispielsweise möglich, durch die Sensoreinheit eine EKG-Messung durchzuführen, wobei durch die Mittel zur Erfassung von Zusatzdaten die Beschleunigung und/oder Lage im Raum des Lebewesens erfaßt wird. Statt der Beschleunigung kann jedoch auch die Atmung oder ein sonstiger physiologischer Parameter des Lebewesens erfaßt werden. Da durch die Bewegung des Lebewesens Artefakte während der EKG-Messung auftreten können, ist es somit vorteilhafterweise möglich, die Artefakte entsprechend zu korrigieren.

Weiterhin bevorzugterweise sind die Mittel zur Erfassung von Zusatzdaten ausgelegt, im wesentlichen die gleichen physiologischen Parameter, welche durch die Sensoreinheit erfasst werden, zu erfassen. Somit wirken Sensoreinheit und Mittel zur Erfassung von Zusatzdaten als im wesentlichen "Mehrfachsensoren", d.h. es werden physiologische Parameter gleicher Art erfasst. Somit ergibt sich vorteilhafterweise eine Redundanz der Messergebnisse, so dass Artefakte vorteilhafterweise erkannt werden können. Beispielsweise wäre es möglich, für die Pulsmessung die doppelte Anzahl an Elektroden zu verwenden oder entsprechend zwei Manschetten für die Blutdruckmessung.

Vorteilhafterweise sind die Mittel zur Erfassung von Zusatzdaten als weitere Sensoreinheit ausgebildet. Es versteht sich somit, dass es auch möglich ist, für eine Art von Parameterdaten eine Vielzahl von Mitteln zur Erfassung von Zusatzdaten vorzusehen, welche einerseits die gleichen physiologischen Parameter und andererseits ebenfalls andere physiologische Parameter erfassen.

In einer bevorzugten Ausführungsform sind die Mittel zur Erfassung von Zusatzdaten ausgelegt, durch die Sensoreinheit erfasste Sekundärparameter zu erfassen. Somit stehen die Mittel zur Erfassung von Zusatzdaten mittelbar oder unmittelbar mit der Sensoreinheit in Signalverbindung, um entsprechend die Sekundärparameter zu erfassen. Infolgedessen werden vorzugsweise "interne" Sensordaten den Mitteln zur Erfassung von Zusatzdaten übermittelt.

Bevorzugterweise erfasst die Sensoreinheit physiologische Parameter in Form einer Stromspannung und Sekundärparameter in Form eines Stromwiderstands. Somit ist vorteilhafterweise möglich, eine zuverlässige EKG-Messung am Lebewesen durchzuführen, wobei die Sensoreinheit das EKG (d.h. die Spannung im/am Lebewesen) erfasst und zusätzlich auch der Kontaktwiderstand zwischen einem Paar von Elektroden als Sekundärparameter erfasst. Infolgedessen ist es vorteilhafterweise möglich, zu erkennen, ob ein Artefakt in der Messung vorliegt oder eine Störung der Messung beispielsweise durch eine nicht korrekte Positionierung der Elektroden.

In einer weiteren bevorzugten Ausführungsform ist die Korrektureinheit ausgelegt, die zu korrigierenden Parameter über ein Korrektursignal zu verändern und/oder über einen Signalfilter herauszufiltern und vorzugsweise zu verwerfen. In anderen Worten werden somit durch die Mittel zur Erfassung von Zusatzdaten Zusatzinformationen gewonnen, um die über die Sensoreinheit ermittelten Parameterdaten zu verwerfen oder zu korrigieren. Die Verarbeitungseinheit, und insbesondere die Korrektureinheit, dient somit als Signalfilter, um die mit Fehlern bzw. Artefakten versehenen Parameterdaten zu erkennen und entsprechend zu verarbeiten.

Weiterhin bevorzugterweise ist die Korrektureinheit ausgelegt, der Sensoreinheit ein Steuerungssignal zu übermitteln, um eine Wiederholung der Erfassung des physiologischen Parameters zu bewirken. Vorteilhafterweise dient die Verarbeitungseinheit, und insbesondere die Korrektureinheit, als Steuerungseinrichtung zur Steuerung der Messung durch die Sensoreinheit, wobei diese eine Wiederholung der Messungen vorzugsweise unmittelbarer zeitlicher Abfolge bewirkt. Somit wird eine vorteilhafterweise zuverlässige und genaue Messung der Parameter gewährleistet.

Vorzugsweise weist die Datenerfassungseinrichtung weiterhin eine Feedbackeinrichtung auf, welche ausgelegt ist, Daten von der Verarbeitungseinheit und/oder einer externen Einrichtung zu empfangen und vorzugsweise über Ausgabemittel auszugeben. Dazu ist es beispielsweise möglich, die Parameterdaten an die externe Einrichtung zu übermitteln, welche entsprechend hiermit in Zusammenhang stehende Daten zurücksendet bzw. übermittelt. Diese können vorteilhafterweise über Ausgabemittel an das Lebewesen ausgegeben werden, wobei die Ausgabemittel akustisch und/oder optisch sein können. Die Feedbackeinrichtung kann jedoch ebenfalls auch von der Verarbeitungseinheit selbst Daten empfangen, wobei dieses insbesondere in Abhängigkeit von den mit Fehlern bzw. Artefakten versehenen zu korrigierenden Parameterdaten stehen. So kann beispielsweise über die Feedbackeinrichtung dem Lebewesen eine Information zugeführt werden, dass eine aktuelle Messung über die Sensoreinheit nicht korrekt ausgeführt wurde und eine entsprechende Handlung verlangt werden (beispielsweise die korrekte Positionierung von Elektroden bei einer EKG-Messung).

Weiterhin erfindungsgemäß ist ein Verfahren zur Erfassung von physiologischen Parametern eines Lebewesens vorgesehen, umfassend folgende Schritte:
- Erfassen zumindest eines physiologischen Parameters eines Lebewesens mittels zumindest einer Sensoreinheit;
- Erzeugen von Parameterdaten aus den physiologischen Parametern durch die Sensoreinheit;
- Erfassen von mit den physiologischen Parametern in Zusammenhang stehenden Zusatzdaten durch Mittel zur Erfassung von Zusatzdaten;
- Vergleichen der Parameterdaten und der Zusatzdaten durch einen Komparator einer Verarbeitungseinheit; und
- Übermitteln eines Signals vom Komparator an eine Korrektureinheit der Verarbeitungseinheit in Abhängigkeit des Vergleichsergebnisses, um eine Korrektur von mit Fehlern versehenen Parameterdaten vorzusehen.

Vorteilhafterweise wird somit ein Verfahren geschaffen, mittels welchem in zuverlässiger und sicherer Art und Weise physiologische Parameter eines Lebewesens erfasst werden können, wobei Artefakte und sonstige Störungen erkannt und vorzugsweise korrigiert oder herausgefiltert werden können.

Die weiteren Merkmale und Vorteile der erfindungsgemäßen Datenerfassungseinrichtung können ebenfalls in dem erfindungsgemäßen Verfahren zur Erfassung von physiologischen Parametern eines Lebewesens Anwendung finden.

Weiterhin erfindungsgemäß ist ein Computerprogrammprodukt vorgesehen, welches Programmteile zur Durchführung des erfindungsgemäßen Verfahrens zur Erfassung von physiologischen Parametern eines Lebewesens umfasst. Somit ist es vorteilhafterweise möglich, das erfindungsgemäße Verfahren auf einem Computer aus- bzw. durchzuführen.

Die Erfindung wird nachfolgend anhand begleitender Zeichnungen bevorzugter Ausführungsformen beispielhaft beschrieben. Es zeigen:
- Fig. 1:: ein schematisches Schaltungsdiagramm einer ersten Ausführungsform der vorliegenden Erfindung,
- Fig. 2:: ein schematisches Schaltungsdiagramm einer zweiten Ausführungsform der vorliegenden Erfindung,
- Fig. 3:: ein schematisches Schaltungsdiagramm einer dritten Ausführungsform der vorliegenden Erfindung.

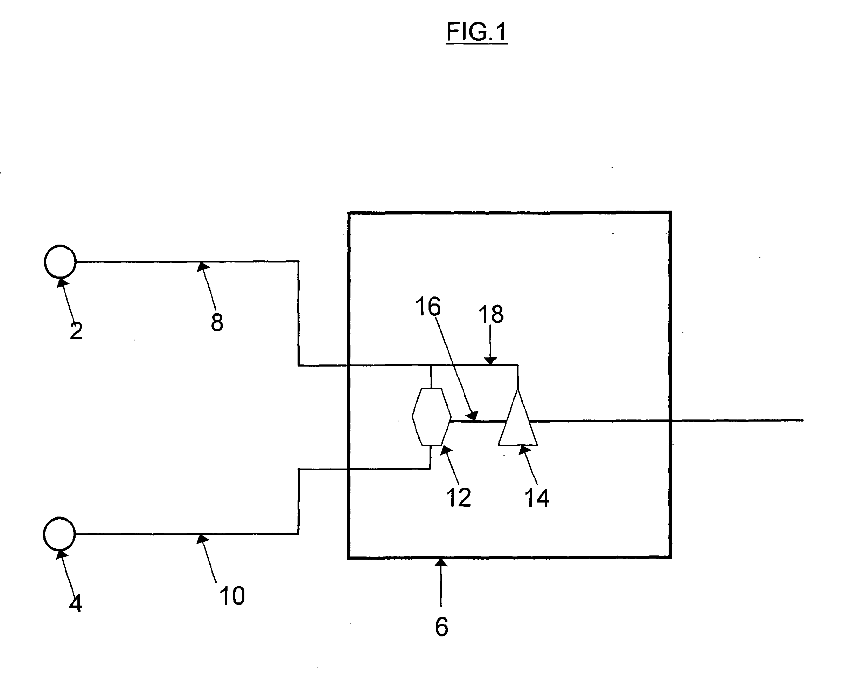

In Fig. 1 ist ein schematisches Schaltungsdiagramm der erfindungsgemäßen Datenerfassungseinrichtung zur Erfassung von physiologischen Parametern eines Lebewesens dargestellt. Die Datenerfassungseinrichtung weist zumindest eine Sensoreinheit 2 und zumindest ein Mittel zur Erfassung von Zusatzdaten 4 auf, welche mit einer Verarbeitungseinheit 6 in Signalverbindung 8,10 stehen.

Die Sensoreinheit 2 ist ausgelegt, zumindest einen physiologischen Parameter des Lebewesens zu erfassen und entsprechende Parameterdaten zu erzeugen. Physiologische Parameter sind insbesondere das EKG, der Puls, der Sauerstoffsättigungsgehalt des Bluts, die Körpertemperatur, die Lage und Bewegung/Beschleunigung zumindest eines Teils des Körpers im Raum, die Hautfeuchte, der Hautwiderstand etc. Die Sensoreinheit 2 erzeugt aus diesem zumindest einen erfassten bzw. gemessenen physiologischen Parameter entsprechende Parameterdaten, welche über eine Signalverbindung 8 an die Verarbeitungseinheit 6 übermittelt werden.

Die Sensoreinheit 2 ist in der dargestellten Ausführungsform von der Verarbeitungseinheit 6 und dem Mittel zur Erfassung von Zusatzdaten 4 getrennt ausgebildet, kann jedoch ebenfalls integral bzw. im wesentlichen einstückig mit der Verarbeitungseinheit 6 und/oder dem Mittel zur Erfassung von Zusatzdaten 4 ausgebildet sein. Zumindest die am Lebewesen angeordneten Einheiten (Sensoreinheit 2, Mittel 4) sind mobil ausgebildet.

Die Mittel zur Erfassung von Zusatzdaten 4 sind vorgesehen, um mit dem zumindest einen physiologischen Parameter in Zusammenhang stehende Zusatzdaten zu erfassen. Diese können einerseits mit dem physiologischen Parameter identisch oder zusätzlich oder alternativ ebenfalls ein weiterer, anderer physiologischer Parameter oder eine sonstige physikalische Messgröße sein. Die Mittel 4 stehen über eine Signalverbindung 10 mit der Verarbeitungseinheit 6 in Signalverbindung, über welche die Zusatzdaten übermittelt werden können.

Die Verarbeitungseinheit 6 umfasst im wesentlichen einen Komparator 12 und eine Korrektureinheit 14. Der Komparator 12 ist ausgelegt, die Zusatzdaten den Parameterdaten zuzuordnen, so dass durch eine Veränderung der Zusatzdaten Rückschlüsse auf entsprechende Änderungen in den Parameterdaten gezogen werden können, insbesondere hinsichtlich veränderter Messbedingungen. Infolgedessen ist es möglich, Fehler oder Artefakte während der Messung durch die Sensoreinheit 2 festzustellen. In Abhängigkeit dieses Vergleichsergebnisses übermittelt der Komparator 12 über eine Signalverbindung 16 ein Signal an die Korrektureinheit 14. Durch dieses Signal wird die Korrektureinheit 14 veranlasst, eine Korrektur der Parameterdaten vorzusehen, welche der Korrektureinheit 14 über die Signalverbindung 18 zugeführt werden. Infolgedessen kann die Korrektureinheit 1 4 als Filter wirken, d.h. ein fehlerbehaftetes bzw. mit Artefakten versehenes Signal zu verwerfen oder als Korrekturmittel dienen, d.h. die Signale bzw. Parameterdaten "wieder-gut-zu-rechnen" und entsprechend auszugeben.

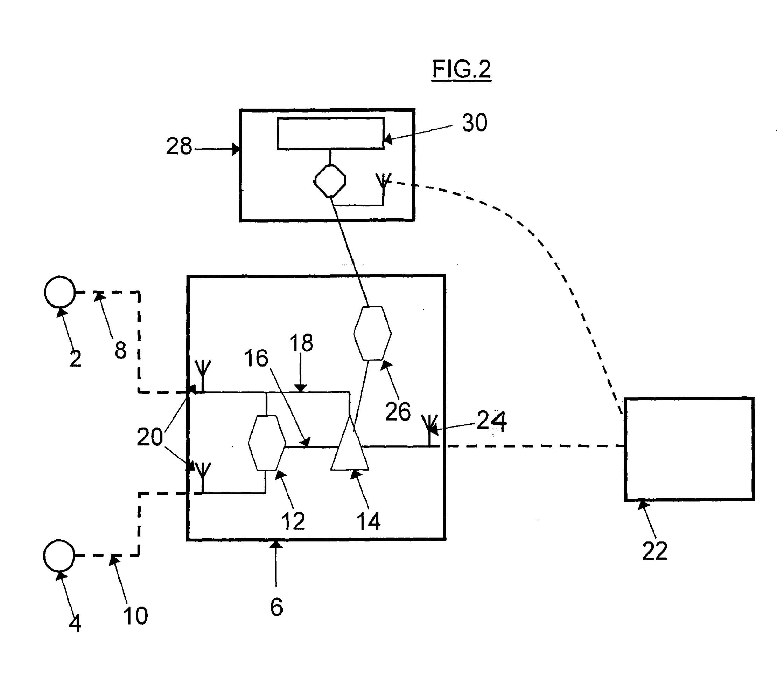

In Fig. 2 ist eine weitere Ausführungsform der vorliegenden Erfindung dargestellt, wobei die mit der ersten Ausführungsform identischen Merkmale mit den gleichen Bezugsziffern versehen sind. Die Sensoreinheit 2 steht in dieser Ausführungsform jedoch mit der Verarbeitungseinrichtung 6 über Funkübertragungsmittel 20 in Signalverbindung, wie auch die Mittel 4 zur Erfassung von Zusatzdaten. Die Verarbeitungseinheit 6 steht mit einer externen Einrichtung 22 ebenfalls vorzugsweise über Funkübertragungsmittel 24 in Signalverbindung. Die Verarbeitungseinheit 6 ist ausgelegt, die Parameterdaten an die externe Einrichtung 22 zu übermitteln. Darüber hinaus ist es ebenfalls möglich, Daten bzw. Informationen, welche mit den Artefakten bzw. Fehlern in Zusammenhang stehen, an diese zu übermitteln. Darüber hinaus ist in der Verarbeitungseinheit 6 ein Prozessierungsmittel 26 vorgesehen, welches mit einer Feedbackeinrichtung 28 in Signalverbindung steht. Die Feedbackeinrichtung 28 umfasst Ausgabemittel 30, um Feedbackinformationen optisch und/oder akustisch auszugeben. Somit lassen sich insbesondere vorteilhafterweise Feedbackinformationen mit Bezug auf die Messung der Parameterdaten übermitteln, beispielsweise ob eine Fehlmessung vorliegt und wie diese behoben werden kann. Zusätzlich oder alternativ kann die Feedbackeinrichtung ebenfalls, vorzugsweise über Funkübertragungsmittel, mit der externen Einrichtung 22 in Signalverbindung stehen, so dass ebenfalls von der externen Einrichtung 22 übermittelte Feedbackinformationen auf den Ausgabemitteln 30 ausgegeben werden können.

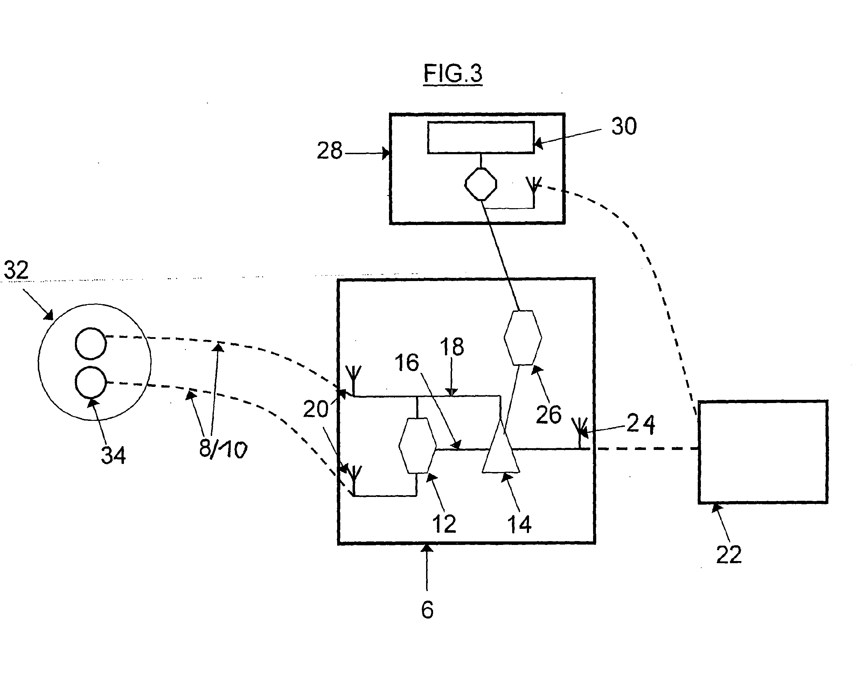

In Fig. 3 ist eine dritte Ausführungsform der vorliegenden Erfindung dargestellt. In der dargestellten Ausführungsform sind Sensoreinheit 2 und die Mittel zur Erfassung der Zusatzdaten 4 im wesentlichen als ein Element 32 ausgebildet. Über einen entsprechenden Sensor, beispielsweise über zwei Elektroden 34, wird zumindest ein physiologischer Parameter des Lebewesens erfasst und entsprechende Parameterdaten erzeugt. Diese Parameterdaten werden über die Signalverbindung 8 für Parameterdaten der Verarbeitungseinheit 6 übermittelt. Die Mittel zur Erfassung von Zusatzdaten sind ebenfalls in dem Element 32 vorgesehen. Diese sind ausgelegt, Sekundärparameter der Sensoreinheit, d.h. der Elektroden 34, zu erfassen. Somit könnte beispielsweise der Widerstand zwischen den zwei Elektroden erfasst werden, welcher über die Signalverbindung 10 der Verarbeitungseinheit 6 übermittelt wird.

Die oben beschriebenen Ausführungsformen der Erfindung können selbstverständlich ebenfalls miteinander kombiniert werden, so dass mehrere Sensoreinheiten 2 und mehrere Mittel 4 vorgesehen werden können. Insbesondere ist es von Vorteil, die Sensoren am Körper des Lebewesens zu verteilen, um eine sichere Erkennung von Artefakten bzw. Fehlern zu ermöglichen.

### Bezugszeichenliste

- 2: Sensoreinheit
- 4: Mittel zur Erfassung von Zusatzdaten
- 6: Verarbeitungseinheit
- 8: Signalverbindung
- 10: Signalverbindung
- 12: Komparator
- 14: Korrektureinheit
- 16: Signalverbindung
- 18: Signalverbindung
- 20: Funkübertragungsmittel
- 22: externe Einrichtung
- 24: Funkübertragungsmittel
- 26: Prozessierungsmittel
- 28: Feedbackeinrichtung
- 30: Ausgabemittel
- 32: Element
- 34: Elektrode

## Patentansprüche

1. Datenerfassungseinrichtung zur Erfassung von physiologischen Parametern eines Lebewesens, umfassend:
- zumindest eine Sensoreinheit (2), welche ausgelegt ist, zumindest einen physiologischen Parameter des Lebewesens zu erfassen und entsprechende Parameterdaten zu erzeugen;
- zumindest ein Mittel bzw. eine Zusatzdatenerfassungseinheit (4), welche ausgelegt ist, mit dem physiologischen Parameter im Zusammenhang stehende Zusatzdaten zu erfassen; und
- zumindest eine Verarbeitungseinheit (6) mit einem Komparator (12) zum Vergleich der Parameterdaten und der Zusatzdaten, wobei der Komparator (12) ausgelegt ist, in Abhängigkeit des Vergleichsergebnisses ein Signal an eine Korrektureinheit (14) der Verarbeitungseinheit (6) zu übermitteln, um eine Korrektur von mit Fehlern versehenen Parameterdaten vorzusehen.

2. Datenerfassungseinrichtung nach Anspruch 1, wobei die Mittel (4) zur Erfassung von Zusatzdaten ausgelegt sind, andere als die durch die Sensoreinheit (2) erfassten Parameter, insbesondere andere physiologische Parameter, zu erfassen.

3. Datenerfassungseinrichtung nach einem der vorhergehenden Ansprüche, wobei die Mittel (4) zur Erfassung von Zusatzdaten ausgelegt sind, im wesentlichen die gleichen physiologischen Parameter, welche durch die Sensoreinheit (2) erfasst werden, zu erfassen.

4. Datenerfassungseinrichtung nach einem der vorhergehenden Ansprüche, wobei die Mittel (4) zur Erfassung von Zusatzdaten als weitere Sensoreinheit ausgebildet sind.

5. Datenerfassungseinrichtung nach einem der vorhergehenden Ansprüche, wobei die Mittel (4) zur Erfassung von Zusatzdaten ausgelegt sind, durch die Sensoreinheit (2) erfasste Sekundärparameter zu erfassen.

6. Datenerfassungseinrichtung nach Anspruch 5, wobei die Sensoreinheit (2) physiologische Parameter in Form einer Stromspannung und Sekundärparameter in Form eines Stromwiderstands erfasst.

7. Datenerfassungseinrichtung nach einem der vorhergehenden Ansprüche, wobei die Korrektureinheit (14) ausgelegt ist, die zu korrigierenden Parameterdaten über ein Korrektursignal zu verändern und/oder über einen Signalfilter herauszufiltern und vorzugsweise zu verwerfen.

8. Datenerfassungseinrichtung nach einem der vorhergehenden Ansprüche, wobei die Korrektureinheit (14) ausgelegt ist, der Sensoreinheit (2) ein Steuerungssignal zu übermitteln, um eine Wiederholung der Erfassung des physiologischen Parameters zu bewirken.

9. Datenerfassungseinrichtung nach einem der vorhergehenden Ansprüche, weiterhin aufweisend eine Feedbackeinrichtung (28), welche ausgelegt ist, Daten von der Verarbeitungseinheit (6) und/oder einer externen Einrichtung (22) zu empfangen und vorzugsweise über Ausgabemittel (30) auszugeben.

10. Verfahren zur Erfassung von physiologischen Parametern eines Lebewesens, umfassend folgende Schritte:
Erfassen zumindest eines physiologischen Parameters eines Lebewesens mittels zumindest einer Sensoreinheit (2);
Erzeugen von Parameterdaten aus den physiologischen Parametern durch die Sensoreinheit (2);
Erfassen von mit den physiologischen Parametern in Zusammenhang stehenden Zusatzdaten durch eine Zusatzdatenerfassungseinheit bzw. Mittel (4) zur Erfassung von Zusatzdaten;
Vergleichen der Parameterdaten und der Zusatzdaten durch einen Komparator (12) einer Verarbeitungseinheit (6);
Übermitteln eines Signals vom Komparator (12) an eine Korrektureinheit (14) der Verarbeitungseinheit (6) in Abhängigkeit des Vergleichsergebnisses, um eine Korrektur von mit Fehlern versehenen Parameterdaten vorzusehen.

11. Computerprogrammprodukt, welches Programmteile zur Durchführung eines Verfahrens nach Anspruch 10 umfaßt.
